**Europäisches Patentamt**

(19) **European Patent Office**

**Office européen des brevets**

(11) Numéro de publication: **0 086 705 B1**

## (12) FASCICULE DE BREVET EUROPEEN

(45) Date de publication du fascicule du brevet:
05.02.86

(51) Int. Cl.⁴: **C 07 J 9/00, A 61 K 9/08, A 61 K 31/575**

(21) Numéro de dépôt: **83400264.4**

(22) Date de dépôt: **08.02.83**

(54) **Solution aqueuse d'acide ursodésoxycholique à usages thérapeutiques, son procédé de préparation et application au traitement des lithiases biliaires.**

(30) Priorité: **12.02.82 FR 8202312**

(43) Date de publication de la demande:
**24.08.83 Bulletin 83/34**

(45) Mention de la délivrance du brevet:
**05.02.86 Bulletin 86/6**

(84) Etats contractants désignés:
**BE DE GB IT LU NL SE**

(56) Documents cités:
**CHEMICAL ABSTRACTS, vol. 78, no. 6, 12 février 1973, page 263, résumé no. 33933v, Columbus, Ohio, USA**
**CHEMICAL ABSTRACTS, vol. 78, no. 6, 12 février 1973, page 262, résumé no. 33924t, Columbus, Ohio, USA**

(73) Titulaire: **S.A. PANMEDICA Société dite:, Zone Industrielle - Ilot J, F-06510 Carros (FR)**

(72) Inventeur: **Laruelle, Claude, Avenue Bellevue, F-06270 Villeneuve Loubet (FR)**
Inventeur: **Lepant, Marcel, l'Escoundu Allée Clairefontaine, F-06140 Vence (FR)**

(74) Mandataire: **Ores, Irène et al, CABINET ORES 6, Avenue de Messine, F-75008 Paris (FR)**

## Description

La présente invention est relative à une solution aqueuse d'acide ursodésoxycholique à usages thérapeutiques, à son procédé de préparation et à son application au trai-tement des lithiases biliaires.

Le traitement pharmaceutique (souvent associé aux moyens diétetiques, crenothérapiques et chirurgicaux), constitue un moyen de choix pour éliminer les calculs de cholestérol formes dans la vésicule biliaire ou dans les voies biliaires. Ce traitement fait surtout appel à des dérivés de l'acide cholanique comme l'acide ursodesoxycholique ou l'acide chénodésoxycholique.

En effet, l'acide ursodésoxycholique présente la propriété tres intéressante de pouvoir dissoudre les calculs de cholestérol ; absorbé par voie générale, il est résorbé dans l'ileon : il transforme la bile saturée en cholestérol (bile lithogène) en une bile insaturee. L'acide ursodésoxycholique favorise donc la dissolution des calculs cholestéroliques en modifiant la composition biliaire.

L'administration par voie orale présente toutefois certains inconvénients. Une mauvaise résorption gastrique et intestinale entraîne la multiplication des prises. La modification des proportions relatives des acides choliques dans la bile n'intervenant que lentement, la dissolution des calculs cholestéroliques est elle-même longue et laborieuse.

La dose thérapeutique journalière par voie orale étant d'environ 500 mg à 1 g en deux ou trois prises, entraîne une élévation assez faible du taux sanguin d'acide ursodésoxycholique ; par contre, l'injection intra-veineuse d'une solution de cet acide entraîne une augmentation rapide de la cholérèse.

Il était donc intéressant de pouvoir disposer d'une forme liquide injectable d'acide ursodésoxy-cholique. Un intérêt supplémentaire de cette forme soluble était la possibilité de percolation d'un calcul biliaire au moyen d'un drain de Kehr dans les cas ou l'intervention chirurgicale sur la vesicule biliaire est incomplete ou limitée voire meme déconseillée, notamment dans le cas de microcalculs pouvant réamorcer la precipitation cholestérique.

L'acide ursodesoxycholique est un acide relative-ment faible, pouvant donner des sels avec les bases minérales fortes telles que la soude, la potasse, entre autres. Comme le signalent H. IGIMI et M.C. CAREY (Journal of Lipid Research 21 - 72 (1980)), l'acide ursodésoxy-cholique précipite de sa solution à 37°C des que le pH atteint 8,0/8,1, alcrs que son épimere l'acide chéno-desoxycholique ne précipite qu'a pH 7,0. La solubilité aqueuse de l'acide ursodésoxycholique est en effet tres faible, de l'ordre de 50 µ/litre.

La réalisation de formes solubles a déjü ete tentée (cf. la préparation de solutés injectables de l'acide ursodésoxycholique revendiquée dans le Brevet japonais 7228123 (Tokyo Tanabe)). Cette réalisation presente l'inconvénient majeur de contenir de grandes quantités de solvants. La solution è 1 % contient 5 ml de diméthylacétamide et 60 ml de propylèneglycol. Cette quantité de solvants est à déconseiller pour des solutions injectables et à proscrire absolument pour une percolation dans la vésicule biliaire. Un autre Brevet japonais (n° 7228124) emploie moins de solvant, mais présente l'inconvénient d'être à pH 9,1 pour une solution à 10 %.

La présente invention s'est par conséquent fixé pour but de pourvoir à des solutions de l'acide urso-désoxycholique qui répondent mieux aux nécessités de la pratique que les solutions de l'Art antérieur, notamment en ce qu'elles sont concentrées, dépourvues totalement de solvant, en ce qu'elles présentent des pH physiologiques acceptables et résistant parfaitement aux traitements de stérilisation.

La présente invention a pour objet une solution aqueuse d'acide ursodésoxycholique à usages thérapeutigues, caractéri-sée en ce qu'elle contient, associée à l'acide ursodésoxy-cholique, de la pipérazine.

Suivant un mode de réalisation particulièrement avantageux de l'objet de l'invention, les quantités de l'acide ursodésoxycholique et de la pipérazine, sont stoechiométriques et forment un sel parfaitement soluble dans l'eau : l'ursodésoxycholate de pipérazine.

La présente invention a également pour objet un procédé de préparation de la solution d'acide urso-désoxycholique, caractérisé en ce que l'on met en suspension l'acide ursodésoxycholique, en ce que l'on ajoute de la pipérazine sous agitation jusqu'a dissolution complete, et en ce que l'on filtre et stérilise la solution ainsi obtenue.

Conformément à l'invention, il est possible ainsi de préparer des solutions aqueuses suffisamment concen-trées d'acide ursodésoxycholique, ne contenant aucun solvant organique présentant un pH physiologique (soit inférieur à 7,5), et résistant parfaitement aux traitements de stérilisation.

Ce procédé permet la solubilisation dans un grand domaine de dilution, pouvant aller de 50 % à 0,1 % et per-met ainsi d'obtenir des formes de l'acide ursodésoxycho-lique convenant parfaitement aussi bien aux injections, ü la percolation intra-cholédocienne qu'aux traitements oraux.

La composition pharmaceutique injectable conforme à la présente invention, peut être utilisée aussi bien en thérapeutique humaine qu'en thérapeutique vétérinaire.

## EXEMPLE

On disperse 78,5 g d'acide ursodésoxycholique (0,2 mole) dans 800 ml d'eau distillée, on agite et on traite par 17,2 g de pipérazine (0,2 mole). Quand la solution est complete, on filtre quelques impuretes sur plaque millipore, on lave avec 2 fois 100 ml d'eau, puis on stérilise par traitement a l'autoclave 30 minutes a 120°C. La solution contient environ 80 g par litre d'acide ursodesoxycholique à un pH de 7,75 et peut etre utilisée directement ou apres dilution.

Les solutions d'acide ursodésoxycholique ainsi obtenues sont tres stables. Ainsi, apres plusieurs essais repetes de stérilisation, il n'est apparu aucune trace de dégradation de l'acide ursodésoxycholique dissous, ni transformation en l'un de ses acides homologues, cholique, lithocholique, chénodésoxycholique et désoxycholique. Ceci a été vérifié par chromatographie en couche mince sur plaque de silice dans les systemes : chloroforme 24, méthanol 3, acide acétique 1 ou cyclohexane 50, acétate d'ethyle 48, acide acétique 2.

Ces formes solubles ne précipitent pas par dilution (meme extrême) avec de l'eau distillée ou avec une solution physiologique de chlorure de sodium. Elles sont stables dans le temps et résistent bien a une température meme élevée. Elles supportent bien l'addition d'antioxydants ou de conservateurs aux doses habituelles.

Les solutions conformes à l'invention ainsi obtenues, peuvent être administrées par voie injectable, par voie orale, et peuvent être percolées in vivo directement sur des calculs cholestéroliques.

## Revendications

1°- Solution aqueuse d'acide ursodésoxycholique à usages thérapeutiques, caractérisée en ce qu'elle contient, associée à l'acide ursodésoxycholique, de la pipérazine, les deux produits sont en quantité stoéchiométrique et for-ment un sel parfaitement soluble dans l'eau, 1,urso-désoxycholate de pipérazine.

2°- Procédé de préparation de la solution de l'acide ursodésoxycholique, caractérisé en ce que l'on met en sus-pension l'acide ursodésoxycholique, en ce que l'on ajoute de la pipérazine sous agitation jusqu'à dissolution complète, et en ce que l'on filtre et stérilise la solution ainsi obtenue.

3°- Médicament administrable par voie orale, caracté-risé en ce qu'il contient une quantité thérapeutique active de la solution selon la Revendication 1, associée à des véhicules pharmaceutiques usuels.

4°- Médicament administrable par voie injectable, caractérisé en ce qu'il contient une quantité thérapeutique active de la solution selon la Revendication 1, associée à des véhicules pharmaceutiques usuels.

5°- Médicament administrable par percolation intra-cholédocienne, caractérisé en ce qu'il contient une quantité thérapeutique active de la solution selon la Revendication 1, associée à des véhicules pharmaceutiques usuels.

## Patentansprüche

1. Wässerige Ursodeoxycholsäure-Lösung für therapeu-tische Anwendungen, dadurch gekennzeichnet, daß sie zusammen mit der Ursodeoxycholsäure Piperazin enthält, wobei die zwei Produkte in stöchiometrischer Men-ge vorliegen und ein in Wasser vollständiges Salz das Piperazinsalz von Ursodeoxycholsäure bilden.

2. Verfahren zur Herstellung der Lösung von Ursodeoxy-cholsäure, dadurch gekennzeichnet, daß man die Ursodeoxycholsäure in Suspension bringt, und daß man unter Rühren Piperazin bis zur vollständigen Auflösung zugibt, und daß man die so erhaltene Lösung filtriert und sterilisiert.

3. Oral verabreichbares Arzneimittel, dadurch gekennzeichnet, daß es eine therapeutisch ak-tive Menge der Lösung nach Anspruch 1 zusammen mit übli-chen pharmazeutischen Trägern enthält.

4. Durch Injektion verabreichbares Arzneimittel, da-durch gekennzeichnet, daß es eine therapeutisch aktive Menge der Lösung nach Anspruch 1 zusammen mit üblichen pharmazeutischen Trägern enthält.

5. Durch intracholedochiale Perkolation verabreich-bares Arzneimittel, dadurch gekennzeichnet, daß es eine therapeutisch aktive Menge der Lösung nach Anspruch 1 zusammen mit üblichen pharmazeutischen Trägern enthält.

## Claims

1. An aqueous solution of ursodeoxycholic acid for therapeutic uses, characterized in that it contains piperazine in association with the ursodeoxycholic acid, and the two products are in stoichiometric quantities and form a perfectly water-soluble salt, piperazine ursodeoxycholate.

2. A process for the preparation of the solution of ursodeoxycholic acid, characterized in that the urso-deoxycholic acid is suspended, in that piperazine is added, with stirring, until a complete solution is obtained, and in that the solution thus obtained is filtered and sterilized.

3. A drug which can be administered orally, charac-terized in that it contains an active therapeutic quantity of the solution according to Claim 1, in association with customary pharmaceutical vehicles.

4. A drug which can be administered by injection, characterized in that it contains an active therapeutic quantity of the solution according to Claim 1, in association with customary pharmaceutical vehicles.

5. A drug which can be administered by intracholedochal diffusion, characterized in that it contains an active therapeutic quantity of the solution according to Claim 1, in association with customary pharmaceutical vehicles.